# FASCICULE DE BREVET EUROPEEN

(11) **EP 1 858 591 B1**
(45) Date de publication et mention de la délivrance du brevet: **18.01.2012**
(21) Numéro de dépôt: 06726251.9
(22) Date de dépôt: 15.03.2006
(51) Int. Cl.: A61N 7/02

(54) **SONDE THERAPEUTIQUE ENDO-CAVITAIRE COMPORTANT UN TRANSDUCTEUR D'IMAGERIE INTEGRE AU SEIN DU TRANSDUCTEUR ULTRASONORE DE THERAPIE**
THERAPEUTISCHE ENDOKAVITÄTEN-SONDE MIT EINEM IM THERAPEUTISCHEN ULTRASCHALLWANDLER INTEGRIERTEN BILDWANDLER
THERAPEUTIC ENDOCAVITY PROBE COMPRISING AN IMAGING TRANSDUCER INTEGRATED WITHIN THE THERAPY ULTRASONIC TRANSDUCER

(30) Priorité: 15.03.2005 FR 0502531
(43) Date de publication de la demande: 28.11.2007
(73) Titulaire: EDAP S.A., 69120 Vaulx en Velin (FR); INSERM (Institut National de la Santé et de la Recherche Médicale), 75654 Paris Cedex 13 (FR)
(72) Inventeur: CHAPELON, Jean-Yves, F-69100 Villeurbanne (FR); CURIEL, Laura, Toronto, ON M4Y 2V6 (CA); MARTIN, Yves, F-01700 Beynost (FR); NALLET, Olivier, F-69003 Lyon (FR)
(74) Mandataire: Thibault, Jean-Marc
(86) Numéro de dépôt international: PCT/FR2006/050229
(87) Numéro de publication internationale: WO 2006/097661

(56) Documents cités:
- EP-A- 0 659 387
- EP-A- 1 034 745
- WO-A-00/72919
- US-A- 5 720 286
- US-A- 5 873 828

## Description

La présente invention concerne une sonde endo-cavitaire de thérapie permettant d'une part le traitement de tissus en particulier de la prostate à l'aide d'ondes acoustiques ultrasonores pulsées et focalisées, et d'autre part la visualisation de ces tissus.

Dans l'état de la technique, il est connu d'utiliser en association avec un transducteur de thérapie émettant des ondes ultrasonores focalisées, un transducteur d'imagerie permettant un suivi en temps réel des tissus.

Dans le domaine des applications extracorporelles, il existe diverses formes de réalisation de sondes avec imagerie. Par exemple, Lizzi et al. dans "'Ultrasonic hyperthermia for ophtalmic therapy' IEEE Trans. Ultrason., Ferroelec., Freq., Contr., 31, pp. 473-481*"* décrivent une sonde échographique pour une application ophtalmologique alors que Vallencien et al. (1996) dans "'Ablation of superficial bladder tumors with focused extracorporeal pyrotherapy' Urology, 47, pp. 204-207*"* et Wu et al. dans "'Pathological changes in human malignant carcinoma treated with high-intensity focused ultrasound', Ultrasound in Med & Biol., 27, pp. 1099-1106*"* proposent des sondes extracorporelles de tailles importantes où la surface occupée par le transducteur d'imagerie est négligeable par rapport à la surface générant les ondes ultrasonores.

Si pour des applications extracorporelles, l'imagerie des tissus ne pose pas de problème particulier, il n'en est pas de même pour une sonde endo-cavitaire telle qu'une sonde endo-rectale, compte tenu des problèmes liés notamment à l'encombrement réduit que doit présenter une telle sonde.

Pour tenter de résoudre ce problème, Vaezy et al. dans "Real-time visualization of high-intensity focused ultrasound treatment using ultrasound imaging", Ultrasound in Med & Biol., vol. 27(1), pp. 33-42, 2001*"* propose une sonde comportant un transducteur d'imagerie situé à l'extrémité d'un transducteur de thérapie. L'inconvénient principal de cette sonde réside dans le fait que l'axe de l'image n'est pas orienté selon l'axe de focalisation du transducteur de thérapie. De plus, la disposition relative des deux transducteurs ne facilite pas son utilisation dans des conditions endo-cavitaires.

Sanghvi et al. dans son brevet US 5 117 832 propose une sonde endo-cavitaire comportant un transducteur de thérapie au centre duquel est placé un transducteur d'imagerie. Pour remédier au problème de la perte de surface du transducteur de thérapie, le transducteur d'imagerie est également utilisé pour la thérapie. Les deux transducteurs ont donc la même fréquence de fonctionnement ou des fréquences de fonctionnement proches qui résultent d'un compromis entre la fréquence de thérapie et celle d'imagerie. Il s'ensuit une qualité d'images altérée et/ou des performances de traitement qui ne sont pas optimales.

De même, la sonde endo-rectale présentée au Congrès ISTU 2002 par Ishida K, Kubota J, Mitake T, Carlson RF, Seip R, Sanghvi NT, Asuma T, Sasaki K, Kawabata K, Umemura S, dans "'Development and animal experiment of variable focusing HIFU system for prostate cancer treatment*"* comporte une sonde d'imagerie de forme rectangulaire logée au centre de la sonde de thérapie. Même si une telle sonde est conçue pour une application endo-rectale, sa taille très importante interdit en pratique une telle application.

EP 0659 387 divulgue une soude avec un transducteur thérapeutique comportant un transducteur d'imagerie.

L'analyse des solutions antérieures connues conduit à constater qu'il apparaît le besoin de disposer d'une sonde dont les dimensions permettent une utilisation endo-cavitaire tout en étant pourvu d'un transducteur apte à réaliser une imagerie en temps réel de bonne qualité, combiné à un transducteur de thérapie dont les performances de traitement ne sont pas affectées par la présence du transducteur d'imagerie.

Pour satisfaire un tel besoin, les déposants ont eu le mérite de rechercher une solution dans l'intégration du transducteur d'imagerie directement au sein du transducteur de thérapie alors même qu'une telle solution conduit de prime abord à une dégradation de la performance thérapeutique. Par ailleurs, il a été constaté que cette dégradation de la performance s'accompagne d'une dispersion de l'énergie acoustique, susceptible de créer des lésions secondaires indésirables. De façon surprenante, il a été trouvé en particulier une géométrie spécifique entre les transducteurs d'imagerie et thérapeutique, qui permet de réaliser une sonde, qui tout en assurant un suivi en temps réel et une efficacité thérapeutique, autorise une utilisation endo-cavitaire.

L'objet de l'invention vise donc à proposer une sonde thérapeutique endo-cavitaire pour le traitement de tissus par l'émission d'ondes ultrasonores focalisées, comportant un support présentant un axe longitudinal et formant un tube de guidage prolongé par une tête de montage pour au moins un élément transducteur de thérapie de surface active émettant des ondes ultrasonores focalisées et présentant une face frontale sphérique de surface totale dans laquelle est aménagée une fenêtre de surface déterminée, pour le montage d'un transducteur d'imagerie, l'élément transducteur possédant un premier diamètre dans la direction perpendiculaire à l'axe longitudinal qui est inférieur à un deuxième diamètre considéré selon l'axe longitudinal. Selon l'invention l'élément transducteur de thérapie présente une face frontale possédant une surface totale égale à 1 500 mm² ± 200 mm², augmentée de la surface de la fenêtre pour le passage du transducteur d'imagerie, le rapport de la surface de la fenêtre sur la surface active de l'élément transducteur de thérapie étant compris entre 0,45 et 0,25.

Par exemple, le premier diamètre de l'élément transducteur de thérapie est compris entre 33 et 40 mm.

Selon un autre aspect de l'invention, l'élément transducteur de thérapie présente une focale géométrique comprise entre 35 et 45 mm.

Selon un autre aspect de l'invention, l'élément transducteur de thérapie émet des ondes ultrasonores avec une durée d'impulsion se situant dans la gamme de 4 à 7 s et avantageusement égale à 6 s, les ondes ultrasonores étant séparées par une durée d'extinction se situant dans la gamme de 4 à 7 s et avantageusement égale à 4 s.

Selon encore un autre aspect de l'invention, l'élément transducteur de thérapie fournit une intensité ultrasonore pic au point focal comprise entre 500 et 3 000 w/cm² et de préférence entre 1 000 et 1 500 w/cm².

De préférence, l'élément transducteur de thérapie possède une fréquence de fonctionnement comprise entre 2 et 5MHz et de préférence égale à 3 MHz.

Selon une caractéristique avantageuse de réalisation, le transducteur d'imagerie est monté sur la tête de manière indépendante par rapport à l'élément transducteur de thérapie.

Selon cet exemple préféré de réalisation, le transducteur d'imagerie est monté de manière amovible sur la tête qui possède une partie avant ouverte pour permettre le positionnement de l'élément transducteur de thérapie, et une partie arrière munie d'un volet pour autoriser l'accès au transducteur d'imagerie.

Dans un exemple de réalisation, le transducteur d'imagerie est monté de manière amovible sur une olive de support sur la tête, s'étendant en saillie par rapport à la face frontale de l'élément transducteur de thérapie.

Dans un exemple de réalisation, le transducteur d'imagerie est monté de manière que le plan image formé par ledit transducteur d'imagerie est transversal ou longitudinal par rapport à l'axe longitudinal de la sonde.

Selon une variante de réalisation, il est à noter que des moyens de déplacement agissent sur l'olive de support en vue d'assurer sa rotation pour permettre au transducteur d'imagerie de former alternativement un plan d'image transversal puis longitudinal.

Il apparaît particulièrement avantageux que le transducteur d'imagerie et l'élément transducteur de thérapie se trouvent montés de manière que l'axe de focalisation de l'élément transducteur de thérapie soit compris dans le plan d'imagerie du transducteur d'imagerie.

De préférence, le transducteur d'imagerie possède une fréquence centrale comprise entre 5 et 10 MHz et de préférence égale à 7,5 MHz.

Selon une forme préférée de réalisation, la tête entoure l'élément transducteur de thérapie par l'intermédiaire d'un bord distal à profil circulaire s'étendant à l'opposé d'un bord proximal faisant partie d'une section tronconique de raccordement au tube de guidage, le bord distal se prolongeant de part et d'autre par des congés de raccordement à des bords longitudinaux à profils circulaires reliés à la section tronconique.

Selon cette variante préférée de réalisation, la section tronconique de raccordement comporte un canal d'amenée d'un liquide de couplage et un canal de récupération du liquide de couplage reliés respectivement à des canalisations d'amenée et de récupération placées à l'intérieur du tube de guidage, chaque canal débouchant à partir du bord proximal au niveau de chaque bord longitudinal.

De préférence, le tube de guidage possède une section droite transversale circulaire et à proximité de la section tronconique de raccordement, des rainures de positionnement et d'étanchéité pour une membrane apte à enfermer la tête de la sonde.

Pour autoriser une introduction endo-cavitaire, l'olive de support du transducteur d'imagerie s'étend en retrait du plan tangent au bord proximal et au bord distal.

Avantageusement, la tête est aménagée de manière que le plan tangent au bord proximal et au bord distal soit convergent en direction du bord distal.

Un autre aspect de l'invention est de proposer un appareil de traitement de tissus comportant une sonde endo-cavitaire de thérapie conforme à l'invention.

Un autre aspect de l'invention est de proposer un appareil de traitement comportant une sonde de type endo-cavitaire pour le traitement des tissus de la prostate.

Selon une autre application, l'objet de l'invention vise à proposer un appareil de traitement comportant une sonde de type endo-vaginale ou coelioscopique.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue d'une sonde thérapeutique endo-cavitaire en position d'introduction ou de traitement.
La **Figure 2** est une vue en coupe partielle longitudinale prise sensiblement selon les lignes II-II de la **Fig. 1**.
La **Figure 3** est une vue en coupe transversale de la sonde endo-cavitaire conforme à l'invention prise sensiblement selon les lignes III-III de la **Fig. 2**.
La **Figure 4** est une vue frontale de l'élément transducteur de thérapie conforme à l'invention.
La **Figure 5** est une vue en perspective partielle de la sonde endo-cavitaire conforme à l'invention.
Les figures **6A** à **6C** représentent un calcul des lésions biologiques obtenues dans les tissus pour différents rapports de la surface de la fenêtre imagerie sur la surface active de l'élément transducteur de thérapie.

L'objet de l'invention concerne une sonde thérapeutique endo-cavitaire 1 faisant partie de manière connue d'un appareil non représenté de traitement de tissus. Dans l'exemple de réalisation illustré sur les Figures, l'objet de l'invention est une sonde endo-cavitaire adaptée pour la destruction de tissus en particulier de la prostate. Bien entendu, l'objet de l'invention peut être mis en oeuvre pour d'autres types de sondes afin de constituer par exemple, une sonde endo-vaginale pour le traitement par exemple des fibromes, ou une sonde coelioscopique.

Dans l'exemple illustré, la sonde endo-cavitaire **1** comporte un support **2** présentant un axe longitudinal **A** et conformé pour présenter de manière classique, une tête **3** prolongée par un tube de guidage **4** relié à un corps principal **5** intégrant les différents moyens liés au fonctionnement de la sonde endo-cavitaire.

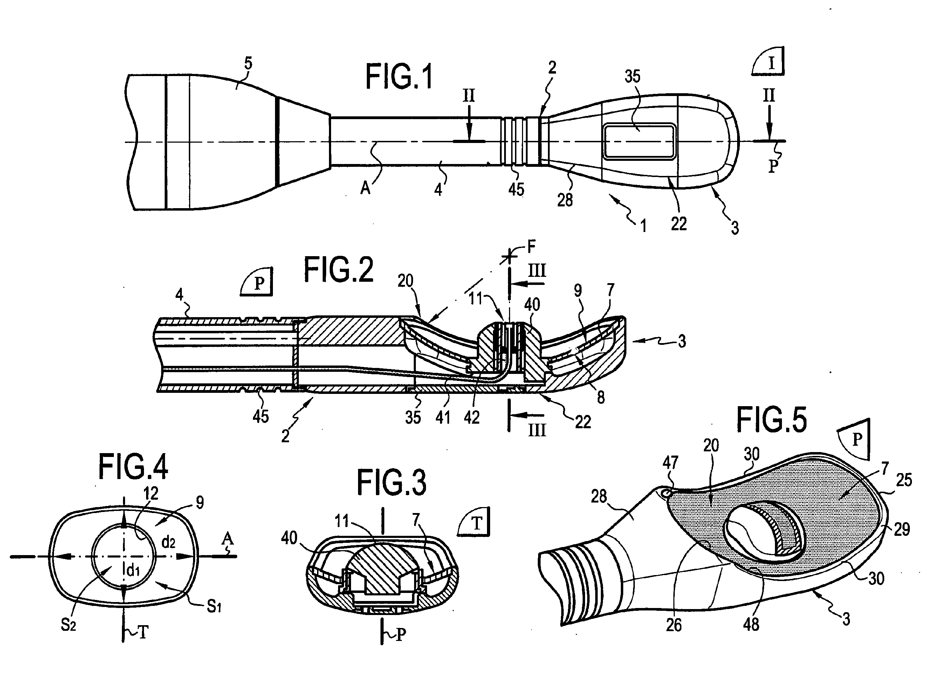

Tel que cela ressort plus précisément des **Fig. 2** à **5**, la sonde endo-cavitaire **1** comporte au moins un élément transducteur de thérapie **7** émettant des ondes ultrasonores focalisées. De manière classique, l'élément transducteur **7** est de préférence choisi parmi le groupe consistant d'une céramique piézo-électrique conventionnelle ou d'une céramique piézo-électrique composite mono-élément ou multi-éléments. L'élément transducteur de thérapie **7** présente à l'opposé d'une face arrière **8**, une face frontale sphérique **9** possédant un premier diamètre **d₁** considéré dans un plan transversal **T** perpendiculaire à l'axe longitudinal **A**, qui est inférieur à un deuxième diamètre **d₂** considéré selon l'axe longitudinal **A**. La face frontale sphérique **9** couvre ainsi une surface totale **S** obtenue à partir de la prise en compte des diamètres **d₁**, **d₂**.

La sonde endo-cavitaire **1** comporte également un transducteur d'imagerie **11** intégré directement à l'intérieur de l'élément transducteur de thérapie **7**. A cet effet, la face frontale sphérique **9** de l'élément transducteur de thérapie **7** est aménagée de manière à comporter une ouverture ou une fenêtre **12** de surface **S₂** adaptée pour le montage du transducteur d'imagerie **11**. Il doit donc être compris que la face frontale sphérique **9** possède une surface totale **S** qui est égale à la somme de la surface **S₂** de la fenêtre **12** et d'une surface active **S₁** correspondant à la partie active du transducteur générant les ondes ultrasonores.

Dans l'exemple de réalisation illustrée, plus particulièrement à la **Fig. 4**, la fenêtre **12** aménagée dans l'élément transducteur de thérapie **7** présente une section circulaire. Avantageusement, la fenêtre **12** est aménagée pour être complètement intégrée à l'intérieur de la face frontale sphérique c'est-à-dire pour être complètement entourée par ledit élément transducteur **7**. La fenêtre **12** se trouve ainsi placée au milieu ou au centre de l'élément transducteur de thérapie **7**. Selon une variante préférée de réalisation, la fenêtre **12** est centrée par rapport à l'élément transducteur de thérapie **7** de manière que l'axe de focalisation **F** de l'élément transducteur de thérapie soit compris dans le plan d'imagerie du transducteur d'imagerie **11**. Bien entendu, il peut être envisagé qu'un décalage existe entre le plan d'imagerie et l'axe focal de l'élément transducteur de thérapie **7**.

Conformément à l'invention, l'élément transducteur de thérapie **7** possède une surface active **S₁** égale à 1 500 mm² ± 200 mm². En d'autres termes, la face frontale sphérique 9 présente une surface totale **S** égale à 1 500 mm² ± 200 mm², augmentée de la surface **S₂** de la fenêtre **12**.

Selon une autre caractéristique de l'objet de l'invention, le rapport de la surface **S₂** de la fenêtre **12** sur la surface active **S₁** de l'élément transducteur de thérapie **7** est compris entre 0,45 et 0,25.

Selon une autre caractéristique de l'invention, le premier diamètre **d₁** de l'élément transducteur de thérapie **7** est compris entre 33 et 40 mm de manière à permettre une introduction intracorporelle.

Selon une autre caractéristique de l'invention, l'élément transducteur de thérapie **7** possède une focale géométrique correspondant au rayon de la face frontale sphérique **9** comprise entre 35 et 45 mm.

L'élément transducteur de thérapie **7** est monté dans la tête de support **3** afin d'être protégé tout en assurant l'émission d'ondes ultrasonores. La tête de support **3** constitue un élément fermé présentant une face avant **20** dans laquelle est aménagée une ouverture dans laquelle peut s'étendre la face frontale sphérique **9** de l'élément transducteur de thérapie **7** autorisant ainsi l'émission des ondes ultrasonores. Il peut être considéré que la face avant **20** de la tête de support **3** s'ouvre ainsi dans un plan d'introduction **1** perpendiculaire au plan transversal **T** et au plan de focalisation **P** passant par l'axe longitudinal **A** et l'axe de focalisation **F** de l'élément transducteur de thérapie **7**.

La tête **3** présente à l'opposé de la face avant **20**, une face arrière **22** prolongée par des bords venant entourer au plus près l'élément transducteur de thérapie **7** selon une forme complémentaire au contour dudit élément **7**. A cet égard, la tête **3** présente ainsi dans le plan d'introduction **I**, un bord distal **25** à profil circulaire s'étendant à l'opposé d'un bord proximal **26** faisant partie d'une section tronconique **28** de raccordement au tube de guidage **4**. Le bord distal **25** se prolonge de part et d'autre, par des congés de raccordement **29** à des bords longitudinaux **30** à profil circulaire reliés à la section tronconique **28**.

Selon une autre caractéristique de l'invention, le transducteur d'imagerie **11** est monté sur la tête **3** de manière indépendante par rapport à l'élément transducteur de thérapie **7**. De préférence, le transducteur d'imagerie **11** est monté de manière amovible par rapport à la tête **3** pour permettre son remplacement. A cet effet, la face arrière **22** de la tête **3** est munie d'un volet **35** dont le retrait permet d'accéder au transducteur d'imagerie **11**.

Dans l'exemple de réalisation illustré, le transducteur d'imagerie **11** est constitué par un réseau courbe d'éléments transducteurs montés avantageusement de manière amovible sur un organe support tel qu'une olive **40** fixée sur la tête **3** par exemple par collage. Dans l'exemple illustré, le plan image formé par le transducteur d'imagerie **11** est transversal, c'est-à-dire qu'il est perpendiculaire à l'axe longitudinal **A** de la sonde. Un tel réseau d'éléments transducteurs est alimenté par un câble **41** placé à l'intérieur d'un canal **42** aménagé dans l'olive et communiquant avec l'intérieur de la tête et du tube de guidage **4** pour permettre le montage dudit câble **41**.

Selon un autre mode de réalisation, il est à noter que l'olive **40** peut être fixée dans une position perpendiculaire à celle précédemment décrite de telle sorte que le plan image formé par le transducteur d'imagerie est orienté selon l'axe longitudinal **A** de la sonde.

Selon un autre mode de réalisation, des moyens de déplacement de tous types agissent sur l'olive **40** en vue d'assurer sa rotation pour permettre au transducteur d'imagerie **11** de former alternativement un plan d'image transversal puis longitudinal.

Dans l'exemple illustré, l'olive de support **40** s'étend en saillie par rapport à la face frontale **9** de l'élément transducteur de thérapie **7**. Cependant l'olive de support **40** du transducteur d'imagerie **11** s'étend en retrait du plan tangent au bord proximal **26** et au bord distal **25**. De préférence, il est à noter que la tête 3 est aménagée de manière que le plan tangent au bord proximal **26** et au bord distal **25** soit convergent en direction du bord distal.

Bien entendu, il peut être envisagé une forme de réalisation dans laquelle le transducteur d'imagerie **11** s'étende sensiblement au niveau, voire en retrait par rapport à la face frontale sphérique **9**.

Selon une autre caractéristique de l'invention, l'élément transducteur de thérapie **7** émet des ondes ultrasonores avec une durée d'impulsion se situant dans la gamme de 1 à 20 s et de préférence entre 4 et 7 s et avantageusement égale à 6 s. De façon complémentaire les ondes ultrasonores sont séparées entre elles par une durée d'extinction comprise entre 1 et 20 s et de préférence entre 4 et 7 s et avantageusement égale à 4 s. Avantageusement, l'élément transducteur de thérapie fournit une intensité ultrasonore pic au point focal (I spatial Peak Temporal Peak) comprise entre 500 et 3 000 w/cm² et de préférence entre 1 000 et 1 500 w/cm².

Il est à noter que l'élément transducteur de thérapie **7** présente une fréquence de fonctionnement comprise entre 2 et 5 MHz et de préférence égale à 3 MHz tandis que le transducteur d'imagerie **11** possède une fréquence centrale comprise entre 5 et 10 MHz et de préférence égale à 7,5 MhZ.

Selon une autre caractéristique de l'invention, la tête **3** de la sonde est destinée à être enfermée dans une membrane non représentée mais connue en soi. A cet effet, le tube de guidage **4** possède une section droite transversale circulaire et présente à proximité de la section tronconique de raccordement **28**, des rainures **45** assurant le montage et le positionnement d'une telle membrane mais également l'étanchéité.

Selon un exemple préféré de réalisation, la section tronconique de raccordement **28** comporte un canal d'amenée **47** d'un liquide de couplage et un canal de récupération **48** du liquide de couplage, reliés respectivement à des canalisations d'amenée et de récupération placées à l'intérieur du tube de guidage **4**. Chaque canal **47**, **48** débouche à partir du bord proximal **26** au niveau de chaque bord longitudinal **30** du support.

Tel que cela ressort de la description qui précède, la sonde endo-cavitaire **1** conforme à l'invention permet d'obtenir une performance thérapeutique équivalente à celle d'un transducteur de thérapie de surface d'émission **S₁** dépourvu de transducteur d'imagerie. Une telle équivalence est obtenue par le choix judicieux de la géométrie relative entre les transducteurs de thérapie **7** et d'imagerie **11**, combiné de préférence à une sélection des paramètres de fonctionnement du transducteur de thérapie **7**. Ainsi, à l'aide d'une sonde équipée d'un élément transducteur de thérapie **7** présentant les caractéristiques conformes à l'invention et émettant des ondes ultrasonores avec une durée d'impulsion égale à 6 s et d'une durée d'extinction égale à 4 s, il peut être obtenu, dans le cadre de la destruction de tissus tumoraux de la prostate, des lésions équivalentes à celles obtenues avec une sonde dépourvue de transducteur d'imagerie.

La sonde conforme à l'invention permet ainsi de maîtriser la dispersion de l'énergie acoustique susceptible de créer des lésions secondaires indésirables. En effet, la présence de la fenêtre **12** au centre de l'élément transducteur de thérapie **7** conduit à l'apparition de lobes d'énergie dits secondaires susceptibles d'intervenir par exemple au niveau du rectum quand une lésion profonde est réalisée dans la prostate. Ces lobes secondaires proviennent de la modification importante de la géométrie de l'élément transducteur de thérapie **7**, due à la fenêtre **12** mais également de la perte de la surface active de l'élément transducteur de thérapie **7**.

Il apparaît que l'importance de ces lobes secondaires augmente en fonction de l'accroissement de la surface **S₂** de la fenêtre **12**. Il ressort ainsi que pour un ratio S_{2/}S₁>0,45, le rapport de la pression acoustique mesurée au niveau par exemple de la paroi rectale sur la pression acoustique mesurée au point focal augmente de manière sensible. Ainsi par exemple, pour un ratio S_{2/}S₁= 1, ce rapport est égal à - 12dB tandis que pour un ratio S₂/S₁=0,45, ce rapport est égal à - 16dB. L'expérience montre que lorsque le rapport de la pression acoustique mesurée au niveau de la paroi rectale sur la pression acoustique mesurée au point focal dépasse le seuil de - 16 dB, le risque d'échauffement devient trop important et peut créer des lésions biologiques irréversibles. En effet, dans cette situation, le différentiel de pression entre le point focal du transducteur de thérapie et la paroi rectale par exemple est insuffisant pour circonscrire la lésion biologique à la zone focale. Il est donc important pour la sécurité du patient de maîtriser la pression au niveau de la paroi rectale et pour se faire, conserver un rapport de surface S₂/S₁ inférieur à 0,45.

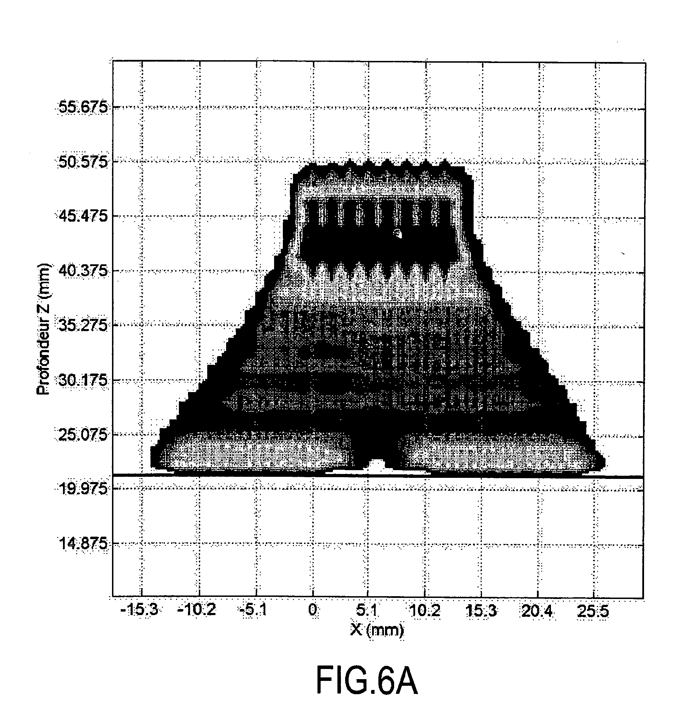

Les figures **6A** à **6C** représentent respectivement la forme de la lésion biologique pour des ratios S₂/S₁ respectivement de 0,37, 0,49 et 1,08.

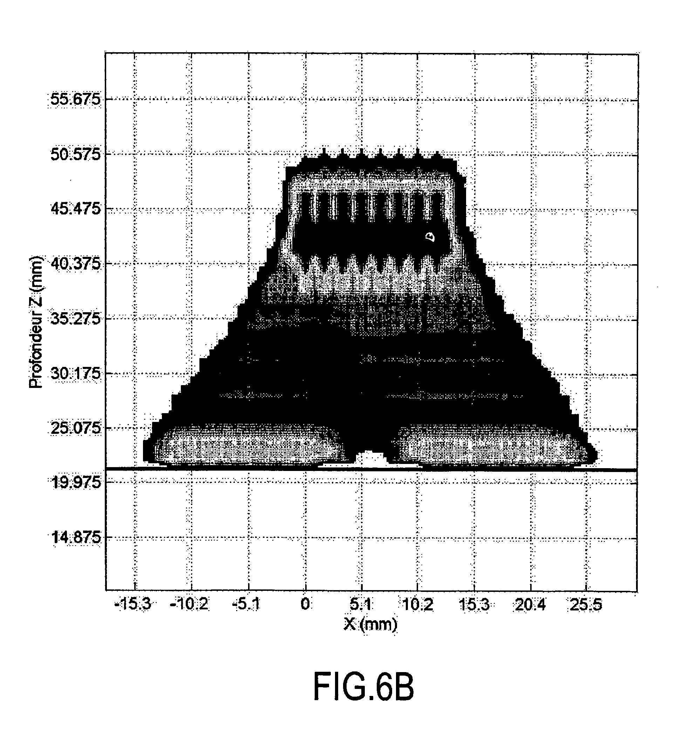

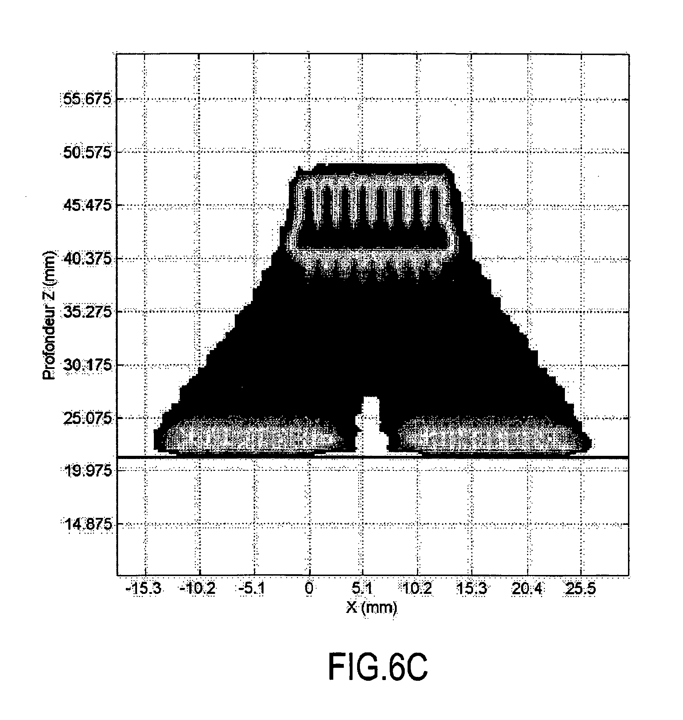

A la figure **6A**, il peut être observé une lésion homogène dans le volume traité. Aux figures **6B** et **6C**, il se forme, au centre et à la base du volume de traitement, une zone non traitée prenant la forme d'une encoche. Il peut être observé aussi une forte diminution de la dose thermique au niveau du point focal (apparition de lésions élémentaires bien différentiées) ainsi que dans le coeur de la zone visée (dose thermique environ deux fois plus faible). Ces figures confirment également l'augmentation de la dose thermique au niveau de la paroi rectale et qui à la figure **6C** dépasse le seuil admissible.

Pour maintenir une dose thermique satisfaisante et un volume de traitement homogène tels que présentée sur la figure **6A**, il faut alors augmenter la puissance acoustique du transducteur. Cette augmentation entraîne une augmentation de la pression et de la dose thermique à la paroi rectale créant une lésion rectale quasi certaine dans le cas d'un rapport S₂/S₁=1,08 et possible dans le cas d'un ratio S₂/S₁=0,49. Ces figures confirment la nécessité de maintenir un ratio S₂/S₁ inférieur ou égal à 0,45.

L'invention n'est pas limitée aux exemples décrits et représentés car diverses modifications peuvent y être apportées sans sortir de son cadre.

## Revendications

1. Sonde thérapeutique endo-cavitaire pour le traitement de tissus par l'émission d'ondes ultrasonores focalisées, comportant un support (**2**) présentant un axe longitudinal (**A**) et formant un tube de guidage (**4**) prolongé par une tête de montage (**3**) pour au moins un élément transducteur de thérapie (**7**) de surface active **S₁** émettant des ondes ultrasonores focalisées et présentant une face frontale (**9**) sphérique de surface totale **S** dans laquelle est aménagée une fenêtre (**12**) de surface **S₂**, pour le montage d'un transducteur d'imagerie (**11**), l'élément transducteur (**7**) possédant un premier diamètre (**d₁**) dans la direction perpendiculaire à l'axe longitudinal qui est inférieur à un deuxième diamètre (**d₂**) considéré selon l'axe longitudinal, **caractérisée en ce que** élément transducteur de thérapie (**7**) présente une face frontale (**9**) possédant une surface totale **S** égale à la somme de la surface active **S₁** égale à 1500 mm² ± 200 mm², et de la surface **S₂** de la fenêtre pour le passage du transducteur d'Imagerie, le rapport de la surface **S₂** de la fenêtre sur la surface active **S₁** de l'élément transducteur de thérapie (7) étant compris entre 0,45 et 0,25.

2. Sonde thérapeutique endo-cavitaire selon la revendication 1, **caractérisée en ce que** la fenêtre (**12**) est aménagée pour être complètement entourée par l'élément transducteur de thérapie (**7**).

3. Sonde thérapeutique endo-cavitaire selon la revendication 1 ou 2, **caractérisée en ce que** le premier diamètre (**d₁**) de l'élément transducteur de thérapie (**7**) est compris entre 33 et 40 mm.

4. Sonde thérapeutique endo-cavitaire selon l'une des revendications 1 à 3, **caractérisée en ce que** l'élément transducteur de thérapie (**7**) émet des ondes ultrasonores avec une durée d'impulsion se situant dans la gamme de 4 à 7 s et avantageusement égale à 6 s, les ondes ultrasonores étant séparées par une durée d'extinction se situant dans la gamme de 4 à 7 s et avantageusement égale à 4 s.

5. Sonde thérapeutique endo-cavitaire selon l'une des revendications 1 à 4, **caractérisée en ce que** l'élément transducteur de thérapie (**7**) fournit une intensité ultrasonore pic au point focal comprise entre 500 et 3 000 w/cm² et de préférence entre 1 000 et 1 500 w/cm².

6. Sonde thérapeutique endo-cavitaire selon l'une des revendications 1 a 5, **caractérisée en ce que** l'élément transducteur de thérapie (**7**) possède une fréquence de fonctionnement comprise entre 2 et 5MHz et de préférence égale à 3 MHz.

7. Sonde thérapeutique endo-cavitaire selon la revendication 1, **caractérisée en ce que** le transducteur d'imagerie (**11**) est monté sur la tête (3) de manière indépendante par rapport à l'élément transducteur de thérapie (**7**).

8. Sonde thérapeutique endo-cavitaire selon la revendication 7, **caractérisée en ce que** le transducteur d'imagerie (**11**) est monté de manière amovible, sur la tête (**3**) qui possède une partie avant ouverte pour permettre le positionnement de l'élément transducteur de thérapie (**7**), et une partie arrière munie d'un volet (**35**) pour autoriser l'accès au transducteur d'imagerie.

9. Sonde thérapeutique endo-cavitaire selon la revendication 8, **caractérisée en ce que** le transducteur d'imagerie (**11**) est monté de manière amovible sur une olive de support (**40**) sur la tête (**3**) s'étendant en saillie par rapport à la face frontale (**9**) de l'élément transducteur de thérapie.

10. Sonde thérapeutique endo-cavitaire selon l'une des revendications 7 à 9, **caractérisée en ce que** le transducteur d'imagerie (**11**) est monté de manière que le plan-image formé par ledit transducteur d'imagerie (**11**) est transversal ou longitudinal par rapport à l'axe longitudinal (**A**) de la sonde.

11. Sonde thérapeutique endo-cavitaire selon les revendications 9 et 10, **caractérisée en ce que** des moyens de déplacement agissent sur l'olive de support (**40**) en vue d'assurer sa rotation pour permettre au transducteur d'imagerie (**11**) de former alternativement un plan d'image transversal puis longitudinal.

12. Sonde thérapeutique endo-cavitaire selon la revendication 1, **caractérisée en ce que** le transducteur d'imagerie (**11**) et l'élément transducteur de thérapie (**7**) sont montés de manière que l'axe de focalisation de l'élément transducteur de thérapie soit compris dans le plan d'imagerie du transducteur d'imagerie.

13. Sonde thérapeutique endo-cavitaire selon la revendication 1, **caractérisée en ce que** le transducteur d'imagerie (**11**) possède une fréquence centrale comprise entre 5 et 10 MHz et de préférence égale à 7,5 MHz.

14. Sonde thérapeutique endo-cavitaire selon la revendication 1, **caractérisée en ce que** la tête (**3**) entoure l'élément transducteur de thérapie (**7**) par l'intermédiaire d'un bord distal (**25**) à profil circulaire s'étendant à l'opposé d'un bord proximal (**26**) faisant partie d'une section tronconique (**28**) de raccordement au tube de guidage (**4**), le bord distal (**25**) se prolongeant de part et d'autre par des congés de raccordement à des bords longitudinaux (**30**) à profils circulaires reliés à la section tronconique (**28**).

15. Sonde thérapeutique endo-cavitaire selon la revendication 14, **caractérisée en ce que** la section tronconique de raccordement (**28**) comporte un canal d'amenée (**47**) d'un liquide de couplage et un canal de récupération (**48**) du liquide de couplage reliés respectivement à des canalisations d'amenée et de récupération placées à l'intérieur du tube de guidage, chaque canal (**47**, **48**) débouchant à partir du bord proximal (**26**) au niveau de chaque bord longitudinal (**30**).

16. Sonde thérapeutique endo-cavitaire selon la revendication 14 ou 15, **caractérisée en ce que** le tube de guidage (**4**) possède une section droite transversale circulaire et à proximité de la section tronconique de raccordement (**28**), des rainures (**45**) de positionnement et d'étanchéité pour une membrane apte à enfermer la tête de la sonde.

17. Sonde thérapeutique endo-cavitaire selon les revendications 9 et 14, **caractérisée en ce que** l'olive de support (**40**) du transducteur d'imagerie (**11**) s'étend en retrait du plan tangent au bord proximal (**26**) et au bord distal (**25**).

18. Sonde thérapeutique endo-cavitaire selon la revendication 17, **caractérisée en ce que** la tête (**3**) est aménagée de manière que le plan tangent au bord proximal (**26**) et au bord distal (**25**) soit convergent en direction du bord distal.

19. Appareil de traitement de tissus **caractérisé en ce qu'**il comprend une sonde endo-cavitaire de thérapie (**1**) telle que définie à l'une quelconque des revendications 1 à 18

20. Appareil de traitement selon la revendication 19, **caractérisé en ce qu'**il comporte une sonde (**1**) de type endo-rectale pour le traitement des tissus de la prostate.

21. Appareil de traitement selon la revendication 19, **caractérisé en ce qu'**il comporte une sonde (**1**) de type endo-vaginale ou coelioscopique.

## Claims

1. A therapeutic endocavity probe for treating tissues by emitting focused ultrasonic waves, including a support (**2**) having a longitudinal axis (**A**) and forming a guide tube (**4**) extended by a mounting head (**3**) for at least one therapy transducer component (**7**) with an active surface area **S₁** emitting focused ultrasonic waves and having a spherical front face (**9**) with a total surface area **S** in which a window (**12**) of surface area **S₂** is laid out, for mounting an imaging transducer (**11**), the transducer component (**7**) having a first diameter (**d₁**) in the direction perpendicular to the longitudinal axis which is smaller than a second diameter (**d₂**) considered along the longitudinal axis, **characterized in that** the therapy transducer component (**7**) has a front face (**9**) having a total surface area **S** equal to the sum of the active surface area **S₁** equal to 1,500 mm² ± 200 mm², and of the surface area **S₂** of the window for letting through the imaging transducer, the ratio of the surface area **S₂** of the window over the active surface area **S₁** of the therapy transducer component (**7**) being included between 0,45 and 0,25.

2. The therapeutic endocavity probe according to claim 1, **characterized in that** the window (**12**) is laid out to be completely surrounded by the therapy transducer component (**7**).

3. The therapeutic endocavity probe according to claim 1 or 2, **characterized in that** the first diameter (**d₁**) of the therapy transducer component (**7**) is between 33 and 40 mm.

4. The therapeutic endocavity probe according to any of claims 1 to 3, **characterized in that** the therapy transducer component (**7**) emits ultrasonic waves with a pulsed duration located in the range from 4 to 7 s and advantageously equal to 6 s, the ultrasonic waves being separated by an extinction duration located in the range from 4 to 7 s, and advantageously equal to 4 s.

5. The therapeutic endocavity probe according to any of claims 1 to 4, **characterized in that** the therapy transducer component (**7**) provides a peak ultrasonic intensity at the focal point between 500 and 3,000 W/cm² and preferably between 1,000 and 1,500 W/cm².

6. The therapeutic endocavity probe according to any of claims 1 to 5, **characterized in that** the therapy transducer component (**7**) has an operating frequency between 2 and 5 MHz and preferably equal to 3 MHz.

7. The therapeutic endocavity probe according to claim 1, **characterized in that** the imaging transducer (**11**) is mounted on the head (**3**) independently of the therapy transducer component (**7**).

8. The therapeutic endocavity probe according to claim 7, **characterized in that** the imaging transducer (**11**) is removably mounted on the head (**3**) which has an open front portion in order to allow positioning of the therapy transducer component (**7**), and a rear portion provided with a flap (**35**) in order to allow access to the imaging transducer.

9. The therapeutic endocavity probe according to claim 8, **characterized in that** the imaging transducer (**11**) is removably mounted on a supporting olive (**40**) on the head (**3**) extending and protruding from the front face (**9**) of the therapy transducer component.

10. The therapeutic endocavity probe according to any of claims 7 to 9, **characterized in that** the imaging transducer (**11**) is mounted so as the image plane formed by said imaging transducer (**11**) is transverse or longitudinal relatively to the longitudinal axis (**A**) of the probe.

11. The therapeutic endocavity probe according to claims 9 and 10, **characterized in that** displacement means act on the supporting olive (**40**) in order to provide its rotation so as to allow the imaging transducer (**11**) to alternately form a transverse and then longitudinal image plane.

12. The therapeutic endocavity probe according to claim 1, **characterized in that** the imaging transducer (**11**) and the therapy transducer component (**7**) are mounted so that the focusing axis of the therapy transducer component is comprised in the imaging plane of the imaging transducer.

13. The therapeutic endocavity probe according to claim 1, **characterized in that** the imaging transducer (**11**) has a simple frequency between 5 and 10 MHz and preferably equal to 7.5 MHz.

14. The therapeutic endocavity probe according to claim 1, **characterized in that** the head (**3**) surrounds the therapy transducer component (**7**) via a distal edge (**25**) with a circular profile extending opposite to a proximal edge (**26**) being part of a frusto-conical section (**28**) for connecting to the guide tube (**4**), the distal edge (**25**) extending on either side by chamfers for connecting to longitudinal edges (**30**) with circular profiles connected to the frusto-conical section (**28**).

15. The therapeutic endocavity probe according to claim 14, **characterized in that** the frusto-conical connecting section (**28**) includes a channel (**47**) for feeding a coupling liquid and a channel (**48**) for recovering the coupling liquid connected to feeding and recovery conduits, respectively, placed inside the guide tube, each channel (**47**, **48**) opening out from the proximal edge (**26**) at each longitudinal edge (**30**).

16. The therapeutic endocavity probe according to claim 14 or 15, **characterized in that** the guide tube (**4**) has a circular transverse cross-section and in proximity to the frusto-conical connecting sections (**28**), grooves (**45**) for positioning and sealing a membrane able to confine the head of the probe.

17. The therapeutic endocavity probe according claims 9 and 14, **characterized in that** the supporting olive (**40**) of the imaging transducer (**11**) extends back from the plane tangent to the proximal edge (**26**) and to the distal edge (**25**).

18. The therapeutic endocavity probe according to claim 17, **characterized in that** the head (**3**) is laid out so that the plane tangent to the proximal edge (**26**) and to the distal edge (**25**) is convergent towards the distal edge.

19. An apparatus for treating tissues **characterized in that** it comprises a therapeutic endocavity probe (**1**) as defined in any of claims 1 to 18.

20. The treatment apparatus according to claim 19, **characterized in that** it includes a probe (**1**) of the endorectal type for treating tissues of the prostate.

21. The treatment apparatus according to claim 19, **characterized in that** it includes a probe (**1**) of the endovaginal or coelioscopic type.

## Patentansprüche

1. Therapeutische endokavitäre Sonde für die Behandlung von Gewebe durch Aussenden gebündelter Ultraschallwellen, umfassend einen Träger (2), der eine Längsachse (A) aufweist und ein Führungsrohr (4) bildet, das durch einen Montagekopf (3) für wenigstens ein Therapie-Wandlerelement (7) mit einer aktiven Fläche S₁ verlängert ist, welches gebündelte Ultraschallwellen aussendet und eine sphärische Stirnseite (9) mit einer Gesamtfläche S aufweist, in der eine Öffnung (12) mit einer Fläche S₂ für die Montage eines Bildwandlers (11) ausgebildet ist, wobei das Wandlerelement (7) einen ersten Durchmesser (d₁) in der Richtung senkrecht zur Längsachse aufweist, der kleiner als ein entlang der Längsachse betrachteter zweiter Durchmesser (d₂) ist, **dadurch gekennzeichnet, daß** das Therapie-Wandlerelement (7) eine Stirnseite (9) aufweist, die eine Gesamtfläche S besitzt, welche gleich der Summe aus der aktiven Fläche S₁ gleich 1.500 mm² ± 200 mm² und aus der Fläche S₂ der Öffnung für den Durchgang des Bildwandlers ist, wobei das Verhältnis der Fläche S₂ der Öffnung zur aktiven Fläche S₁ des Therapie-Wandlerelements (7) zwischen 0,45 und 0,25 liegt.

2. Therapeutische endokavitäre Sonde nach Anspruch 1, **dadurch gekennzeichnet, daß** die Öffnung (12) ausgebildet ist, um von dem Therapie-Wandlerelement (7) vollständig umgeben zu werden.

3. Therapeutische endokavitäre Sonde nach Anspruch 1 oder 2, **dadurch gekennzeichnet, daß** der erste Durchmesser (d₁) des Therapie-Wandlerelements (7) zwischen 33 und 40 mm liegt.

4. Therapeutische endokavitäre Sonde nach einem der Ansprüche 1 bis 3, **dadurch gekennzeichnet, daß** das Therapie-Wandlerelement (7) Ultraschallwellen mit einer Pulsdauer aussendet, die im Bereich von 4 bis 7 s liegt und vorteilhafterweise gleich 6 s beträgt, wobei die Ultraschallwellen durch eine Abklingdauer, die im Bereich zwischen 4 und 7 s liegt und vorteilhafterweise gleich 4 s beträgt, getrennt sind.

5. Therapeutische endokavitäre Sonde nach einem der Ansprüche 1 bis 4, **dadurch gekennzeichnet, daß** das Therapie-Wandlerelement (7) eine Spitzenultraschallintensität im Fokus zwischen 500 und 3.000 w/cm² und vorzugsweise zwischen 1.000 und 1.500 w/cm² liefert.

6. Therapeutische endokavitäre Sonde nach einem der Ansprüche 1 bis 5, **dadurch gekennzeichnet, daß** das Therapie-Wandlerelement (7) eine Betriebsfrequenz im Bereich zwischen 2 und 5 MHz und vorzugsweise gleich 3 MHz aufweist.

7. Therapeutische endokavitäre Sonde nach Anspruch 1, **dadurch gekennzeichnet, daß** der Bildwandler (11) an dem Kopf (3) von dem Therapie-Wandlerelement (7) unabhängig angebracht ist.

8. Therapeutische endokavitäre Sonde nach Anspruch 7, **dadurch gekennzeichnet, daß** der Bildwandler (11) an dem Kopf (3), der ein offenes vorderes Teil besitzt, um das Positionieren des Therapie-Wandlerelements (7) zu ermöglichen, sowie ein hinteres Teil, das mit einer Klappe (35) ausgestattet ist, um den Zugang zu dem Bildwandler zuzulassen, lösbar angebracht ist.

9. Therapeutische endokavitäre Sonde nach Anspruch 8, **dadurch gekennzeichnet, daß** der Bildwandler (11) an dem Kopf (3) an einer Halteolive (40), die gegenüber der Stirnseite (9) des Therapie-Wandlerelements vorspringend verläuft, lösbar angebracht ist.

10. Therapeutische endokavitäre Sonde nach einem der Ansprüche 7 bis 9, **dadurch gekennzeichnet, daß** der Bildwandler (11) derart angebracht ist, daß die durch den Bildwandler (11) gebildete Bildebene gegenüber der Längsachse (A) der Sonde quer oder längs verläuft.

11. Therapeutische endokavitäre Sonde nach den Ansprüchen 9 und 10, **dadurch gekennzeichnet, daß** Bewegungsmittel auf die Halteolive (40) wirken, um deren Drehen sicherzustellen, um dem Bildwandler (11) zu ermöglichen, abwechselnd eine Querbildebene dann eine Längsbildebene zu bilden.

12. Therapeutische endokavitäre Sonde nach Anspruch 1, **dadurch gekennzeichnet, daß** der Bildwandler (11) und das Therapie-Wandlerelement (7) derart angebracht sind, daß die Fokussierungsachse des Therapie-Wandlerelements in der Bildebene des Bildwandlers enthalten ist.

13. Therapeutische endokavitäre Sonde nach Anspruch 1, **dadurch gekennzeichnet, daß** der Bildwandler (11) eine Mittenfrequenz zwischen 5 und 10 MHz und vorzugsweise gleich 7,5 MHz besitzt.

14. Therapeutische endokavitäre Sonde nach Anspruch 1, **dadurch gekennzeichnet, daß** der Kopf (3) das Therapie-Wandlerelement (7) mittels eines distalen Randes (25) mit kreisförmigem Profil, der gegenüber einem proximalen Rand (26) verläuft, welcher Teil eines kegelstumpfförmigen Abschnitts (28) zum Anschluß an das Führungsrohr (4) ist, umgibt, wobei der distale Rand (25) auf beiden Seiten durch Abrundungen zum Anschluß an Längsränder (30) mit kreisförmigen Profilen, die mit dem kegelstumpfförmigen Abschnitt (28) verbunden sind, fortgesetzt ist.

15. Therapeutische endokavitäre Sonde nach Anspruch 14, **dadurch gekennzeichnet, daß** der kegelstumpfförmige Anschlußabschnitt (28) einen Kanal zum Zuführen (47) einer Koppelflüssigkeit sowie einen Kanal zum Rückführen (48) der Koppelflüssigkeit umfaßt, die mit Zuführ- bzw. Rückführleitungen verbunden sind, welche innerhalb des Führungsrohrs angeordnet sind, wobei jeder Kanal (47, 48) aus dem proximalen Rand (26) im Bereich eines jeden Längsrandes (30) ausmündet.

16. Therapeutische endokavitäre Sonde nach Anspruch 14 oder 15, **dadurch gekennzeichnet, daß** das Führungsrohr (4) einen kreisförmigen Querschnitt aufweist sowie in der Nähe des kegelstumpfförmigen Anschlußabschnitts (28) Nuten (45) zum Positionieren und Abdichten für eine Membran, die geeignet ist, den Kopf der Sonde zu umschließen, besitzt.

17. Therapeutische endokavitäre Sonde nach den Ansprüchen 9 und 14, **dadurch gekennzeichnet, daß** die Halteolive (40) des Bildwandlers (11) gegenüber der Tangentialebene an den proximalen Rand (26) und an den distalen Rand (25) vertieft liegt.

18. Therapeutische endokavitäre Sonde nach Anspruch 17, **dadurch gekennzeichnet, daß** der Kopf (3) derart ausgebildet ist, daß die Tangentialebene an den proximalen Rand (26) und an den distalen Rand (25) in Richtung des distalen Randes konvergierend ist.

19. Gerät zur Gewebebehandlung, **dadurch gekennzeichnet, daß** es eine therapeutische endokavitäre Sonde (1), wie sie in einem der Ansprüche 1 bis 18 definiert ist, umfaßt.

20. Behandlungsgerät nach Anspruch 19, **dadurch gekennzeichnet, daß** es eine Sonde (1) vom endorektalen Typ für die Behandlung der Gewebe der Prostata umfaßt.

21. Behandlungsgerät nach Anspruch 19, **dadurch gekennzeichnet, daß** es eine Sonde (1) vom endovaginalen oder zölioskopischen Typ umfaßt.
